# FASCICULE DE BREVET EUROPEEN

(11) **EP 3 694 589 B1**
(45) Date de publication et mention de la délivrance du brevet: **08.12.2021**
(21) Numéro de dépôt: 18812226.1
(22) Date de dépôt: 09.10.2018
(51) Int. Cl.: A61M 15/00, A61M 11/02, B05B 11/06

(54) **DISPOSITIF DE DISTRIBUTION DE PRODUIT FLUIDE**
FLUIDVERTEILERVORRICHTUNG
FLUID DISTRIBUTION APPARATUS

(30) Priorité: 12.10.2017 FR 1759549
(43) Date de publication de la demande: 19.08.2020
(73) Titulaire: Aptar France SAS, 27110 Le Neubourg (FR)
(72) Inventeur: POULLAIN, Franck, 27400 La Haye Malherbe (FR)
(74) Mandataire: CAPRI
(86) Numéro de dépôt international: PCT/FR2018/052492
(87) Numéro de publication internationale: WO 2019/073165

(56) Documents cités:
- WO-A1-02/45866
- GB-A- 1 436 028
- US-A- 4 534 343
- US-A1- 2005 258 273
- US-A1- 2007 060 868

## Description

La présente invention concerne un dispositif de distribution de produit fluide, et plus particulièrement un dispositif pour distribuer unitairement une dose de produit, notamment de poudre, contenue dans un réservoir à l'aide d'un écoulement d'air sous pression.

Le document WO9946055 divulgue un tel dispositif dans lequel un élément de fermeture sphérique, qui obture la sortie du réservoir, est expulsé par l'écoulement d'air créé par une chasse d'air. Pour l'utilisation d'un dispositif de distribution de poudre plus particulièrement, la pression d'air nécessaire pour actionner le dispositif devra être suffisamment élevée pour garantir la distribution complète de la dose, ainsi que son fractionnement si cela est nécessaire. Dans le dispositif susmentionné, la pression d'air nécessaire pour actionner le dispositif est déterminée par la résistance donnée par la bille pour être expulsée. Cette résistance est relativement difficile à contrôler et à prédéterminer puisqu'elle est dépendante du frottement entre la bille et son siège cylindrique dans lequel elle est emmanchée pour obturer de manière étanche ledit réservoir. Il peut par conséquent être nécessaire de minimiser l'interférence entre la sphère et son siège cylindrique, ce qui peut évidemment altérer l'efficacité de l'obturation. De plus, il peut être nécessaire de minimiser la profondeur et le positionnement de la sphère dans son siège afin de faciliter son expulsion. Il peut également être nécessaire de fournir une pression d'air relativement élevée qui n'est pas toujours facile à réaliser à l'aide d'un système de pompe ou d'un système de soufflet, notamment lorsque ces chasses d'air sont actionnées manuellement par le patient. De plus, la distribution, c'est à dire l'expulsion de la bille de son siège, peut se produire à des longueurs différentes de la course de la pompe ou du soufflet de la chasse d'air, de sorte que l'instant précis de la distribution du produit ne peut pas être toujours prédéterminé de manière exacte. Enfin, il y a une limitation dans le choix des matériaux pour la sphère et pour son siège.

Le document WO0245866 décrit un dispositif dans lequel une bille de fermeture est expulsée mécaniquement par une tige solidaire d'une chasse d'air. Ce dispositif n'est pas réutilisable, et l'ensemble du dispositif doit être jeté après son utilisation. Notamment pour des raisons écologiques et économiques, il peut être souhaitable d'avoir un dispositif réutilisable dans lequel le réservoir serait changé après chaque actionnement mais pas la chasse d'air.

Le document WO2015001269 décrit un dispositif similaire à celui du document WO0245866, dans lequel la chasse d'air du dispositif peut être réutilisée avec plusieurs réservoirs de poudre. Ce dispositif présente également des inconvénients. Ainsi, il permet de réutiliser la chasse d'air, mais pas la tête de distribution qui est jetable avec le réservoir après chaque utilisation. De plus, ce dispositif requiert une pièce mobile reliée au réservoir par des ponts sécables, ce qui rend complexe la fabrication du dispositif. Ceci génère de plus un son lors de l'actionnement, lorsque les ponts sécables se brisent, qui peut induire l'utilisateur en erreur. Par ailleurs, les dispositifs des documents WO2015001269 et WO0245866 comportent une tige qui traverse le réservoir, ce qui limite le volume utile de celui-ci et rend le remplissage du réservoir plus complexe.

Le document GB1436028 décrit un dispositif de distribution à capsule dans lequel la capsule est insérée par le haut puis percée lors du vissage de l'embout, la pointe de perçage étant formée sur un organe mobile qui sert uniquement à expulser axialement la capsule vide.

Les documents US2007060868, US4534343 et US2005258273 décrivent d'autres dispositifs de l'état de la technique.

La présente invention a pour but de fournir un dispositif de distribution de produit fluide qui ne reproduit pas les inconvénients susmentionnés.

La présente invention a notamment pour but de fournir un tel dispositif de distribution de produit fluide qui puisse être réutilisé plusieurs fois avec plusieurs réservoirs différents.

La présente invention a aussi pour but de fournir un dispositif de distribution de produit fluide, qui soit simple et peu coûteux à fabriquer, à assembler, à remplir et à utiliser.

La présente a donc pour objet un dispositif de distribution de produit fluide comportant une tête de distribution pourvue d'un orifice de distribution, une chasse d'air pour générer un écoulement d'air lors de l'actionnement du dispositif, et un réservoir contenant une dose unique de produit, ledit réservoir comportant une extrémité axiale proximale et une extrémité axiale distale et étant monté de manière amovible dans ladite tête de distribution, de sorte qu'après l'actionnement du dispositif, le réservoir vide peut être retiré dudit dispositif et remplacé par un nouveau réservoir plein, ladite chasse d'air étant adaptée à revenir en position de repos pour permettre un nouvel actionnement avec ledit nouveau réservoir plein, ladite tête de distribution comportant une pointe de perçage proximale adaptée à percer ladite extrémité axiale proximale dudit réservoir et ledit dispositif comportant un organe de perçage mobile disposé coulissant axialement autour de ladite tête de distribution entre une position de chargement et une position d'actionnement, ledit organe de perçage mobile comportant une pointe de perçage distale adaptée à percer l'extrémité axiale distale dudit réservoir lorsque ledit organe de perçage mobile est déplacé dans sa position d'actionnement.

Avantageusement, ledit réservoir est symétrique.

Avantageusement, ledit réservoir est une capsule ou une gélule.

Avantageusement, lesdites pointes de perçage proximale et distale sont creuses et comportent chacune au moins une ouverture axiale.

Avantageusement, ladite pointe de perçage distale forme une entrée d'air dans ledit réservoir, ladite entrée d'air étant reliée à ladite chasse d'air, et ladite pointe de perçage proximale forme une sortie de produit dans ledit réservoir, ladite sortie de produit étant reliée à ladite ouverture de distribution, de sorte que lors de l'actionnement du dispositif, l'écoulement d'air généré par la chasse d'air pénètre dans ledit réservoir à travers ladite entrée d'air et entraine ledit produit fluide hors dudit réservoir à travers ladite sortie de produit, en direction dudit orifice de distribution.

Avantageusement, ladite extrémité axiale proximale dudit réservoir est percée par ladite pointe de perçage proximale lors de l'insertion dudit réservoir dans ladite tête de distribution.

Avantageusement, ladite tête de distribution comporte une fenêtre latérale, disposée à proximité de ladite pointe de perçage proximale, pour permettre l'insertion dudit réservoir dans ladite tête de distribution.

Avantageusement, ladite tête de distribution comporte une partie mobile ou déformable, telle qu'une lame élastique, opposée à ladite fenêtre latérale, pour expulser le réservoir vide hors de ladite tête de distribution à travers ladite fenêtre latérale.

Avantageusement, ladite chasse d'air comporte un piston coulissant dans une chambre d'air entre une position de repos et une position de distribution, ladite chasse d'air comportant un clapet de sortie permettant lors de l'actionnement de comprimer de l'air dans ladite chambre d'air, un ressort de rappel étant prévu pour solliciter élastiquement ledit piston vers sa position de repos.

Avantageusement, ledit piston, en position de repos, coopère de manière non étanche avec ladite chambre d'air, de sorte que ladite chambre d'air est reliée à l'atmosphère en position de repos.

Selon une première variante avantageuse, ledit clapet de sortie de la chasse d'air comporte une valve passant d'une position de fermeture à une position d'ouverture à partir d'un seuil de pression d'air prédéterminé dans ladite chambre d'air.

Avantageusement, ladite valve comporte une membrane fendue fermée de manière étanche en position de fermeture et qui, à partir d'un seuil de pression prédéterminé, se déforme pour ouvrir ladite fente.

Avantageusement, ladite membrane est convexe en position de fermeture en direction de ladite chambre d'air et lorsque le seuil de pression est atteint, elle inverse sa concavité et devient concave, permettant ainsi l'ouverture de la fente.

Selon une seconde variante avantageuse, ledit clapet de sortie de la chasse d'air comporte une bille déplacée d'une position de fermeture vers une position d'ouverture à partir d'une course d'actionnement prédéterminée dudit piston dans ladite chambre d'air.

Avantageusement, après une partie prédéterminée de la course d'actionnement dudit piston, une tige centrale solidaire dudit piston coopère avec ladite bille pour l'expulser mécaniquement de sa position de fermeture.

Avantageusement, ledit organe de perçage mobile, lorsqu'il est ramené de sa position d'actionnement vers sa position de chargement, coopère avec ladite bille pour la ramener en position de fermeture.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective partiellement découpée d'un dispositif de distribution de produit fluide ou pulvérulent selon un premier mode de réalisation avantageux, en position de repos,
- la figure 2 est une vue schématique en section transversale du dispositif de la figure 1, en cours d'insertion d'une capsule,
- les figures 3 à 8 sont des vues similaires à celle de la figure 2, montrant différentes phases de l'actionnement,
- la figure 9 est une vue schématique de détail en perspective partiellement découpée des moyens de perçage du dispositif de la figure 1,
- les figures 10a et 10b sont des vues schématiques en perspective respectivement de la tête de distribution et de l'organe de perçage mobile de la figure 9,
- les figures 11 à 15 sont des vues schématiques partielles en perspective découpée des différentes phases d'assemblage du dispositif de la figure 1,
- la figure 16 est une vue similaire à celle de la figure 1, montrant un second mode de réalisation avantageux, et
- les figures 17 à 23 sont des vues similaires à celles des figures 2 à 8 du dispositif de la figure 16.

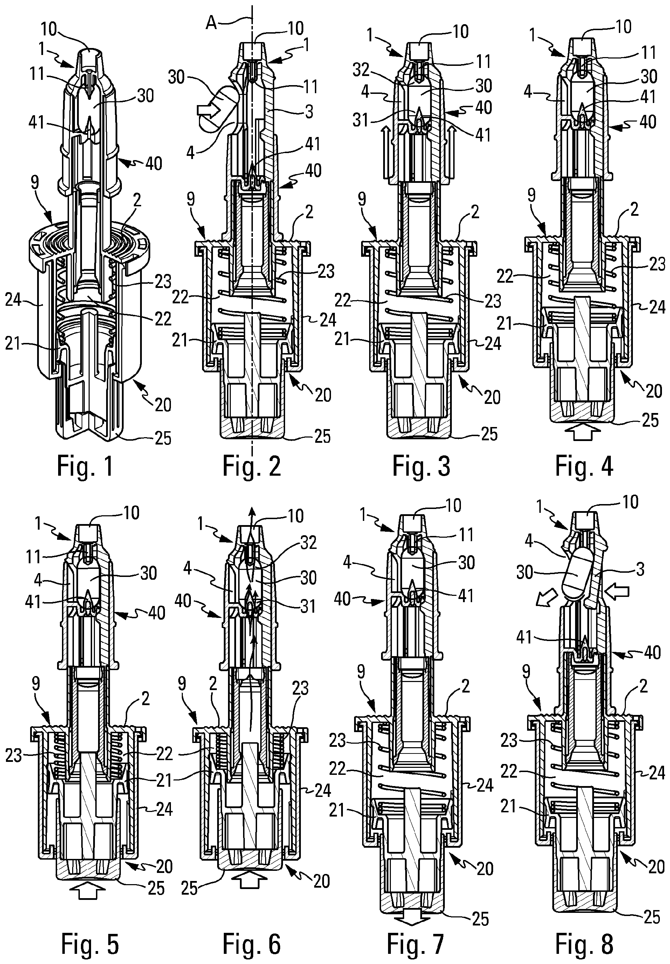

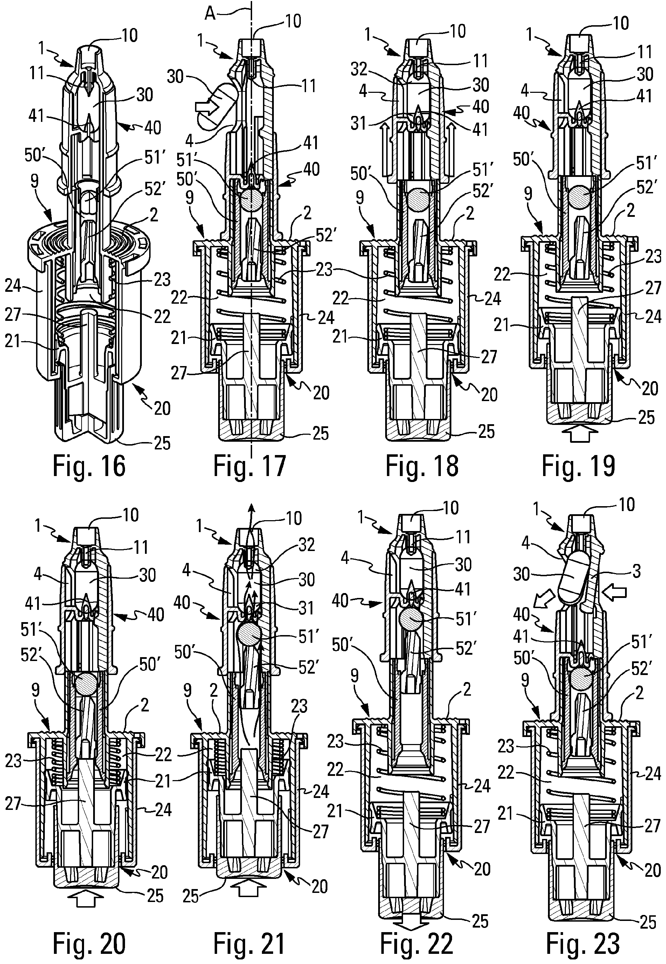

Il est entendu que dans la description ci-après, les termes "supérieur", "inférieur", "haut" et "bas" se réfèrent à la position droite du dispositif représenté notamment sur les figures 1 à 8 et 11 à 23. Les termes "axial" et "radial" se réfèrent à l'axe central longitudinal A du dispositif, représenté sur les figures 2 et 17. Les termes "proximal" et "distal" se réfèrent à l'orifice de distribution.

Le dispositif comporte un réservoir 30 contenant une dose de produit fluide à distribuer, en particulier un médicament sous forme de poudre. Ledit réservoir 30 est de préférence réalisé sous la forme d'une capsule ou gélule étanche de forme axialement allongée. Le réservoir 30 est destiné à être percé en utilisation à ses deux extrémités axiales pour former d'une part une entrée d'air 31 à son extrémité axiale distale et d'autre part une sortie de produit 32 à son extrémité axiale proximale. L'entrée d'air 31 est reliée à une chasse d'air 20 et la sortie de produit 32 est reliée à un orifice de distribution 10 du dispositif. Avant l'actionnement du dispositif, la dose de produit à distribuer est donc maintenue de manière étanche dans ladite capsule 30.

Avantageusement, comme visible sur les figures 1 à 9 et 16 à 23, le réservoir 30 est symétrique, de sorte que le réservoir peut être utilisé dans ses deux orientations axiales.

Le dispositif comporte une chasse d'air 20 qui est actionnée manuellement par l'utilisateur et qui est adaptée à créer un écoulement d'air qui va traverser le réservoir 30 pour emmener le produit qu'il contient en direction de la sortie de distribution 10.

Le réservoir 30 est inséré, notamment emboité, dans une tête de distribution 1 qui comporte l'orifice de distribution 10.

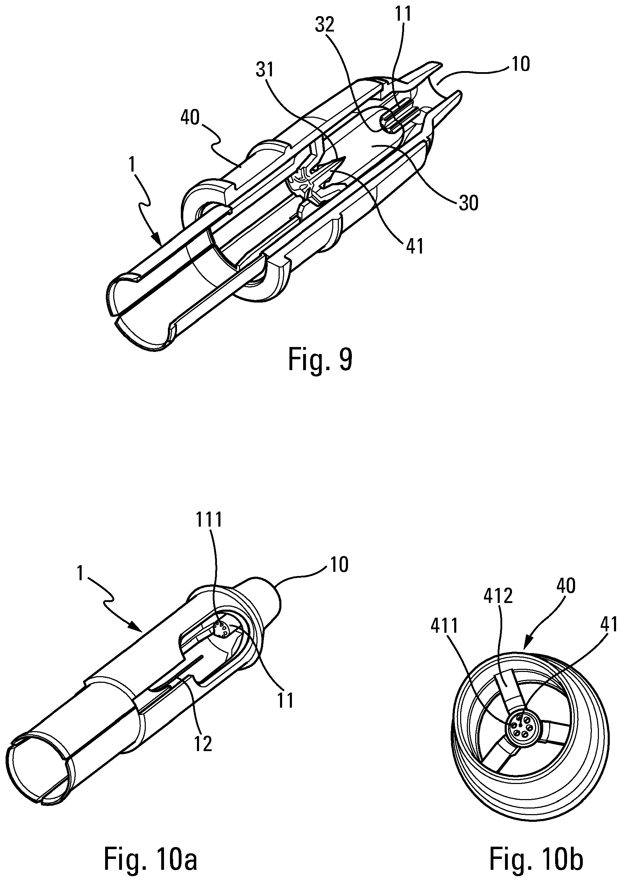

Ladite tête de distribution 1 comporte une pointe de perçage proximale 11 adaptée à percer l'extrémité axiale proximale du réservoir 30 pour former la sortie de produit 32. Cette pointe de perçage proximale 11, visible notamment sur la figure 10a, est creuse et comporte au moins une, de préférence plusieurs ouvertures axiales 111, disposées de préférence autour de la périphérie de ladite pointe. Ainsi, lorsque ladite pointe de perçage proximale 11 pénètre dans le réservoir 30, le produit contenu dans ledit réservoir 30 peut passer à travers lesdites ouvertures axiales 111 pour être expulsé à travers l'orifice de distribution 10.

Ladite tête de distribution 1 comporte une fenêtre latérale 4, disposée à proximité de ladite pointe de perçage proximale 11, pour permettre l'insertion du réservoir 30 dans ladite tête de distribution, comme illustré sur les figures 2 et 17. Avantageusement, cette insertion provoque le perçage de l'extrémité axiale proximale du réservoir 30 par ladite pointe de perçage proximale 11.

Ladite tête de distribution 1 comporte plusieurs fentes longitudinales 12, notamment trois, qui sont notamment visibles sur la figure 10a et dont la fonction sera expliquée ci-après.

La tête de distribution 1 est de préférence réalisée en une pièce monobloc, notamment par moulage.

Un organe de perçage mobile 40 est disposé coulissant axialement autour de ladite tête de distribution 1 entre une position de chargement et une position d'actionnement. Cet organe de perçage mobile 40 comporte une pointe de perçage distale 41 adaptée à percer l'extrémité axiale distale du réservoir 30 pour former l'entrée d'air 31. Cette pointe de perçage distale 41, visible notamment sur la figure 10b, est également creuse et comporte au moins une, de préférence plusieurs ouvertures axiales 411, disposées de préférence autour de la périphérie de ladite pointe. Ainsi, lorsque ladite pointe de perçage distale 41 pénètre dans le réservoir 30, l'air comprimé contenu dans ladite chasse d'air 20 peut passer à travers lesdites ouvertures axiales 411 pour entrainer le produit contenu dans le réservoir 30 vers ladite ouverture de distribution 10.

La pointe de perçage distale 41 est disposée centralement à l'intérieur de l'organe de perçage mobile 40, en étant fixé au corps dudit organe de perçage mobile 40 par plusieurs, notamment trois, entretoises 412, qui sont notamment visibles sur la figure 10b. ces entretoises 412 sont adaptées à coulisser axialement dans les fentes longitudinales 12 correspondantes de la tête de distribution 1.

En position de chargement, ledit organe de perçage mobile 40 n'obstrue pas ladite fenêtre latérale 4 de ladite tête de distribution 1, comme visible sur les figures 2, 8, 17 et 23. En position d'actionnement, visible sur les figures 1, 3 à 7, 16 et 18 à 22, ledit organe de perçage mobile 40 a coulissé axialement vers le haut autour de la tête de distribution 1, avec pour effet d'une part d'obstruer ladite fenêtre latérale 4 et d'autre part de percer l'extrémité axiale distale du réservoir 30 avec ladite pointe de perçage distale 41, comme visible notamment sur les figures 3 et 18. Dans cette position, les deux extrémités axiales du réservoir 30 sont donc percées par les pointes de perçage proximale 11 et distale 41, et le dispositif est prêt à être actionné.

La chasse d'air 20 représentée sur les figures comporte un piston 21 coulissant dans une chambre d'air 22 entre une position de repos et une position de distribution, le piston 21 étant actionné manuellement par l'utilisateur. Avantageusement, cet actionnement est réalisé au moyen d'un élément poussoir 25 assemblé sur ou solidaire dudit piston 21. Un ressort de rappel 23 sollicite élastiquement ledit piston 21, et donc aussi l'élément poussoir 25, vers leur position de repos.

La chambre d'air 22 est formée dans un corps inférieur 9 par ailleurs pourvu d'un repose-doigts 2. Ce repose-doigt comporte des moyens de fixation, notamment par encliquetage, d'un manchon 24 formant butée anti-arrachement pour le piston 21 et l'élément poussoir 25.

En position de repos, visible sur les figures 1, 2, 8, 16, 17 et 23, la chasse d'air 20 est avantageusement ouverte sur l'atmosphère.

Un premier mode de réalisation est décrit sur les figures 1 à 8. Dans ce mode de réalisation, un ensemble de valve 5 est prévu pour permettre la compression de l'air contenu dans la chambre d'air 22 en début d'actionnement. Cet ensemble de valve 5 comporte un corps central 50 dans lequel est assemblé une valve 51 formant clapet de la chasse d'air 20. Cette valve 51 comporte avantageusement une membrane fendue fermée de manière étanche en position de fermeture et qui, à partir d'un seuil de pression prédéterminé va se déformer pour ouvrir ladite fente. Dans l'exemple représenté, la membrane est convexe en position de fermeture en direction de la chambre d'air 22 et lorsque le seuil de pression est atteint, elle inverse sa concavité et devient concave, permettant ainsi l'ouverture de la fente. Cette valve 51 est retenue dans le corps central 50 par un élément de blocage 52 approprié.

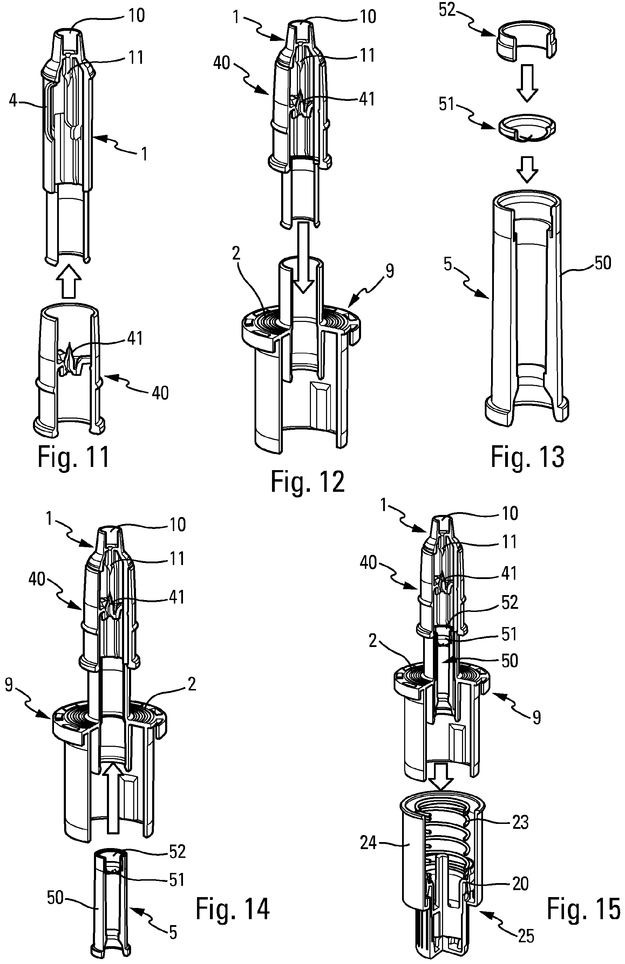

Les figures 11 à 15 illustrent un assemblage avantageux du dispositif des figures 1 à 8. Ainsi, comme visible sur la figure 11, l'organe de perçage mobile 40 est d'abord assemblé autour de la tête de distribution 1. Puis, comme visible sur la figure 12, ce sous-ensemble est monté sur le corps inférieur 9. La figure 13 illustre l'assemblage de l'ensemble de valve 5, à savoir l'insertion de la valve 51 et de son élément de blocage 52 dans le corps central 50. La figure 14 illustre l'assemblage de cet ensemble de valve 5 dans l'unité formée du corps inférieur 9 et de la tête de distribution 1. Enfin, la figure 15 montre l'assemblage final de la chasse d'air 20, avec le piston 21, l'élément poussoir 25 et le ressort de rappel 23.

L'actionnement du dispositif selon le premier mode de réalisation est illustré à titre d'exemple sur les figures 2 à 8.

Ainsi, lorsque l'utilisateur souhaite actionner le dispositif, il place d'une part ses doigts sur le repose-doigt 2 du corps inférieur 9 et d'autre part son pouce sur l'élément poussoir 25, et il exerce une force d'actionnement, qui va déplacer le piston 21 vers sa position de distribution. Dès le début de l'actionnement, visible sur la figure 4, le piston 21 de la chasse d'air va coopérer de manière étanche avec la chambre d'air 22, de sorte que l'air contenu dans ladite chambre d'air 22 va être progressivement comprimé au cours de l'actionnement.

Lorsque le seuil d'ouverture de la valve 51 est atteint, ladite valve s'ouvre, permettant ainsi à l'air comprimé dans la chambre d'air 22 de s'écouler à travers ladite valve 51 et à travers la pointe de perçage distale 41 jusque dans le réservoir 30, pour entrainer le produit fluide contenu dans le réservoir vers l'orifice de distribution 10.

Une course d'actionnement complète va donc expulser la totalité de la dose de produit contenue dans le réservoir 30 au moyen du flux d'air comprimé crée par la chasse d'air 20, comme visible sur la figure 6.

Lorsque l'utilisateur relâche la pression sur l'élément poussoir 25, le ressort de rappel va ramener le piston 21 et l'élément poussoir 25 dans leur position de repos. Lors de cette course de retour, de l'air peut être aspiré dans la chambre d'air 22 à travers la valve 51.

Les figures 16 à 23 illustrent un second mode de réalisation avantageux. Celui-ci est très similaire au premier mode de réalisation décrit ci-dessus, et n'en diffère que par le clapet de la chasse d'air 20.

Dans ce second mode de réalisation, la valve 51 est remplacée par une bille 51'. Cette bille 51' fait partie d'un ensemble de bille 5' comportant un corps central 50' dans lequel est inséré en force ladite bille 51'. Un organe de poussée 52', de préférence creux, peut être monté coulissant dans ledit corps central 50'.

L'assemblage du dispositif selon ce second mode de réalisation est très similaire à celui décrit en référence aux figures 11 à 15, avec la différence que l'ensemble de valve 5 du premier mode de réalisation est remplacé ici par l'ensemble de bille 5'.

L'actionnement du dispositif selon le second mode de réalisation est illustré à titre d'exemple sur les figures 17 à 23, et est très similaire à celui du premier mode de réalisation décrit précédemment en référence aux figures 2 à 8.

La différence concerne l'ouverture du clapet de la chasse d'air 20, ici formé par la bille 51'. Comme visible sur la figure 20, après une partie de la course d'actionnement du piston 21, et donc compression de l'air contenu dans la chambre d'air 22, une tige centrale 27 solidaire du piston 21 et/ou de l'élément poussoir 25 vient coopérer avec l'organe de poussée 52'. Une poursuite de l'actionnement va alors déplacer ledit organe de poussée 52' axialement vers le haut dans ledit corps central 50', pour expulser mécaniquement la bille 51' hors dudit corps central 50', comme visible sur la figure 21. Dans cette variante, ce n'est donc pas la pression de l'air comprimé dans la chambre d'air 22 qui ouvre le clapet de sortie de la chasse d'air 20, mais c'est une ouverture mécanique de ce clapet de sortie qui est réalisée à partir d'une course d'actionnement prédéterminée du piston 21. L'air comprimé peut alors pénétrer dans le réservoir 30 pour expulser son contenu. Lorsque l'organe de perçage mobile 40 est ramené vers sa position de chargement, il va ramener la bille 51' et l'organe de poussée 52' dans leurs positions de départ, à l'intérieur du corps central 50', comme visible sur la figure 23. Il est à noter que l'organe de poussée 52' n'est pas indispensable, et que la tige centrale 27 pourrait coopérer directement avec la bille 51' pour l'expulser.

La présente invention dans toutes ses variantes concerne un dispositif rechargeable.

Ainsi, après actionnement, l'utilisateur peut retirer le réservoir 30 vide. Avantageusement, ce retrait peut être réalisé au moyen d'une partie mobile ou déformable 3 de la tête de distribution 1, telle qu'une lame élastique, opposée à ladite fenêtre latérale 4. Comme visible sur les figures 8 et 23, un appui latéral sur ladite partie mobile ou déformable 3 va pousser le réservoir vide hors de la tête de distribution 1, à travers ladite fenêtre latérale 4. Après retrait du réservoir vide, un nouveau réservoir 30 plein peut être inséré dans la tête de distribution 1 lors du prochain actionnement.

Un avantage de la présente invention est donc la réutilisation de la totalité du dispositif. Seul le réservoir 30 est remplacé et jeté après chaque actionnement.

La présente invention a été décrite en référence à plusieurs modes de réalisation, mais il est entendu qu'un homme du métier peut y apporter toutes modifications, sans sortir du cadre de la présente invention tel que défini par les revendications annexées.

## Revendications

1. Dispositif de distribution de produit fluide comportant une tête de distribution (1) pourvue d'un orifice de distribution (10), une chasse d'air (20) pour générer un écoulement d'air lors de l'actionnement du dispositif, et un réservoir (30) contenant une dose unique de produit, ledit réservoir (30) comportant une extrémité axiale proximale et une extrémité axiale distale et étant monté de manière amovible dans ladite tête de distribution (1), de sorte qu'après l'actionnement du dispositif, le réservoir vide peut être retiré dudit dispositif et remplacé par un nouveau réservoir plein, ladite chasse d'air (20) étant adaptée à revenir en position de repos pour permettre un nouvel actionnement avec ledit nouveau réservoir plein, **caractérisé en ce que** ladite tête de distribution (1) comporte une pointe de perçage proximale (11) adaptée à percer ladite extrémité axiale proximale dudit réservoir (30) et **en ce que** ledit dispositif comporte un organe de perçage mobile (40) disposé coulissant axialement autour de ladite tête de distribution (1) entre une position de chargement et une position d'actionnement, ledit organe de perçage mobile (40) comportant une pointe de perçage distale (41) adaptée à percer l'extrémité axiale distale dudit réservoir (30) lorsque ledit organe de perçage mobile (40) est déplacé dans sa position d'actionnement.

2. Dispositif selon la revendication 1, dans lequel ledit réservoir (30) est symétrique.

3. Dispositif selon la revendication 1 ou 2, dans lequel ledit réservoir (30) est une capsule ou une gélule.

4. Dispositif selon l'une quelconque des revendications précédentes, dans lequel lesdites pointes de perçage proximale (11) et distale (41) sont creuses et comportent chacune au moins une ouverture axiale (111, 411).

5. Dispositif selon l'une quelconque des revendications précédentes, dans lequel ladite pointe de perçage distale (41) forme une entrée d'air (31) dans ledit réservoir (30), ladite entrée d'air (31) étant reliée à ladite chasse d'air (20), et ladite pointe de perçage proximale (11) forme une sortie de produit (32) dans ledit réservoir (30), ladite sortie de produit (32) étant reliée à ladite ouverture de distribution (10), de sorte que lors de l'actionnement du dispositif, l'écoulement d'air généré par la chasse d'air (20) pénètre dans ledit réservoir (30) à travers ladite entrée d'air (31) et entraine ledit produit fluide hors dudit réservoir (30) à travers ladite sortie de produit (32), en direction dudit orifice de distribution (10).

6. Dispositif selon l'une quelconque des revendications précédentes, dans lequel ladite extrémité axiale proximale dudit réservoir (30) est percée par ladite pointe de perçage proximale (11) lors de l'insertion dudit réservoir (30) dans ladite tête de distribution (1).

7. Dispositif selon l'une quelconque des revendications précédentes, dans lequel ladite tête de distribution (1) comporte une fenêtre latérale (4), disposée à proximité de ladite pointe de perçage proximale (11), pour permettre l'insertion dudit réservoir (30) dans ladite tête de distribution (1).

8. Dispositif selon la revendication 7, dans lequel ladite tête de distribution (1) comporte une partie mobile ou déformable (3), telle qu'une lame élastique, opposée à ladite fenêtre latérale (4), pour expulser le réservoir vide hors de ladite tête de distribution (1) à travers ladite fenêtre latérale (4).

9. Dispositif selon l'une quelconque des revendications précédentes, dans lequel ladite chasse d'air comporte un piston (21) coulissant dans une chambre d'air (22) entre une position de repos et une position de distribution, ladite chasse d'air (20) comportant un clapet de sortie (51 ; 51') permettant lors de l'actionnement de comprimer de l'air dans ladite chambre d'air (22), un ressort de rappel (23) étant prévu pour solliciter élastiquement ledit piston (21) vers sa position de repos.

10. Dispositif selon la revendication 9, dans lequel ledit piston (21), en position de repos, coopère de manière non étanche avec ladite chambre d'air (22), de sorte que ladite chambre d'air (22) est reliée à l'atmosphère en position de repos.

11. Dispositif selon la revendication 9 ou 10, dans lequel ledit clapet de sortie de la chasse d'air (20) comporte une valve (51) passant d'une position de fermeture à une position d'ouverture à partir d'un seuil de pression d'air prédéterminé dans ladite chambre d'air (22).

12. Dispositif selon la revendication 11, dans lequel ladite valve (51) comporte une membrane fendue fermée de manière étanche en position de fermeture et qui, à partir d'un seuil de pression prédéterminé, se déforme pour ouvrir ladite fente.

13. Dispositif selon la revendication 12, dans lequel ladite membrane est convexe en position de fermeture en direction de ladite chambre d'air (22) et lorsque le seuil de pression est atteint, elle inverse sa concavité et devient concave, permettant ainsi l'ouverture de la fente.

14. Dispositif selon la revendication 9 ou 10, dans lequel ledit clapet de sortie de la chasse d'air (20) comporte une bille (51') déplacée d'une position de fermeture vers une position d'ouverture à partir d'une course d'actionnement prédéterminée dudit piston (21) dans ladite chambre d'air (22).

15. Dispositif selon la revendication 14, dans lequel, après une partie prédéterminée de la course d'actionnement dudit piston (21), une tige centrale (27) solidaire dudit piston (21) coopère avec ladite bille (51') pour l'expulser mécaniquement de sa position de fermeture.

16. Dispositif selon la revendication 15, dans lequel ledit organe de perçage mobile (40), lorsqu'il est ramené de sa position d'actionnement vers sa position de chargement, coopère avec ladite bille (51') pour la ramener en position de fermeture.

## Patentansprüche

1. Fluidprodukt-Verteilungsvorrichtung, die einen Verteilungskopf (1), der mit einer Verteilungsöffnung (10) versehen ist, eine Luftstoßeinrichtung (20), um bei der Betätigung der Vorrichtung einen Luftstrom zu erzeugen, und einen Vorratsbehälter (30), der eine einzige Dosis des Produkts enthält, umfasst, wobei der Vorratsbehälter (30) ein proximales axiales Ende und ein distales axiales Ende aufweist und in dem Verteilungskopf (1) entnehmbar montiert ist, derart, dass nach der Betätigung der Vorrichtung der leere Vorratsbehälter aus der Vorrichtung gezogen und durch einen neuen, vollen Vorratsbehälter ersetzt werden kann, wobei die Luftstoßeinrichtung (20) dafür ausgelegt ist, in die Ruheposition zurückzukehren, um eine neue Betätigung mit dem neuen, vollen Vorratsbehälter zuzulassen, **dadurch gekennzeichnet, dass** der Verteilungskopf (1) eine proximale Durchstechspitze (11) aufweist, die dafür ausgelegt ist, das proximale axiale Ende des Vorratsbehälters (30) zu durchstechen, und dass die Vorrichtung ein bewegliches Durchstechorgan (40) aufweist, das um den Verteilungskopf (1) axial gleitend zwischen einer gespannten Position und einer Betätigungsposition angeordnet ist, wobei das bewegliche Durchstechorgan (40) eine distale Durchstechspitze (41) aufweist, die dafür ausgelegt ist, das distale axiale Ende des Vorratsbehälters (30) zu durchstechen, wenn das bewegliche Durchstechorgan (40) in seine Betätigungsposition verlagert wird.

2. Vorrichtung nach Anspruch 1, wobei der Vorratsbehälter (30) symmetrisch ist.

3. Vorrichtung nach Anspruch 1 oder 2, wobei der Vorratsbehälter (30) eine Patrone oder eine Kapsel ist.

4. Vorrichtung nach einem der vorhergehenden Ansprüche, wobei die proximale (11) und die distale (41) Durchstechspitze hohl sind und jeweils wenigstens eine axiale Öffnung (111, 411) aufweisen.

5. Vorrichtung nach einem der vorhergehenden Ansprüche, wobei die distale Durchstechspitze (41) einen Lufteingang (31) in den Vorratsbehälter (30) bildet, wobei der Lufteingang (31) mit der Luftstoßeinrichtung (20) verbunden ist, und die proximale Durchstechspitze (11) einen Produktausgang (32) in den Vorratsbehälter (30) bildet, wobei der Produktausgang (32) mit der Verteilungsöffnung (10) verbunden ist, derart, dass bei der Betätigung der Vorrichtung der durch die Luftstoßeinrichtung (20) erzeugte Luftstrom durch den Lufteingang (31) in den Vorratsbehälter (30) eindringt und das Fluidprodukt durch den Produktausgang (32) aus dem Vorratsbehälter (30) in Richtung der Verteilungsöffnung (10) treibt.

6. Vorrichtung nach einem der vorhergehenden Ansprüche, wobei das proximale axiale Ende des Vorratsbehälters (30) von der proximalen Durchstechspitze (11) durchstochen wird, wenn der Vorratsbehälter (30) in den Verteilungskopf (1) eingesetzt wird.

7. Vorrichtung nach einem der vorhergehenden Ansprüche, wobei der Verteilungskopf (1) ein seitliches Fenster (4) aufweist, das in der Nähe der proximalen Durchstechspitze (11) angeordnet ist, um das Einsetzen des Vorratsbehälters (30) in den Verteilungskopf (1) zuzulassen.

8. Vorrichtung nach Anspruch 7, wobei der Verteilungskopf (1) einen beweglichen oder verformbaren Teil (3) wie etwa ein elastisches Plättchen gegenüber dem seitlichen Fenster (4) aufweist, um den leeren Vorratsbehälter durch das seitliche Fenster (4) aus dem Verteilungskopf (1) auszustoßen.

9. Vorrichtung nach einem der vorhergehenden Ansprüche, wobei die Luftstoßeinrichtung einen Kolben (21) aufweist, der in einer Luftkammer (22) zwischen einer Ruheposition und einer Verteilungsposition gleitet, wobei die Luftstoßeinrichtung (20) eine Ausgangsventilklappe (51; 51') aufweist, die bei der Betätigung ermöglicht, Luft in der Luftkammer (22) zu komprimieren, wobei eine Rückstellfeder (23) vorgesehen ist, um den Kolben (21) elastisch in seine Ruheposition zu drängen.

10. Vorrichtung nach Anspruch 9, wobei der Kolben (21) in seiner Ruheposition auf nicht dichte Weise mit der Luftkammer (22) zusammenwirkt, derart, dass die Luftkammer (22) in der Ruheposition mit der Atmosphäre in Verbindung steht.

11. Vorrichtung nach Anspruch 9 oder 10, wobei die Ausgangsventilklappe der Luftstoßeinrichtung (20) ein Ventil (21) aufweist, das ab einem vorgegebenen Luftdruckschwellenwert in der Luftkammer (22) von einer geschlossenen Position in eine geöffnete Position übergeht.

12. Vorrichtung nach Anspruch 11, wobei das Ventil (51) eine geschlitzte Membran aufweist, die in der geschlossenen Position dicht verschlossen ist und sich ab einem vorgegebenen Druckschwellenwert verformt, um den Schlitz zu öffnen.

13. Vorrichtung nach Anspruch 12, wobei die Membran in der geschlossenen Position in Richtung der Luftkammer (22) konvex ist und dann, wenn der Druckschwellenwert erreicht ist, ihre Konkavität umkehrt und konkav ist, womit die Öffnung des Schlitzes ermöglicht wird.

14. Vorrichtung nach Anspruch 9 oder 10, wobei die Ausgangsventilklappe der Luftstoßeinrichtung (20) eine Kugel (51') aufweist, die ab einem vorgegebenen Betätigungshub des Kolbens (21) in der Luftkammer (22) aus einer geschlossenen Position in eine geöffnete Position verlagert wird.

15. Vorrichtung nach Anspruch 14, wobei nach einem vorgegebenen Teil des Betätigungshubs des Kolbens (21) ein mittiger Stift (27), der mit dem Kolben (21) fest verbunden ist, mit der Kugel (51') zusammenwirkt, um sie aus ihrer geschlossenen Position mechanisch auszustoßen.

16. Vorrichtung nach Anspruch 15, wobei das bewegliche Durchstechorgan (40) dann, wenn es aus seiner Betätigungsposition in seine gespannte Position zurückgeführt wird, mit der Kugel (51') zusammenwirkt, um sie in die geschlossene Position zurückzuführen.

## Claims

1. A fluid dispenser device comprising a dispenser head (1) provided with a dispenser orifice (10), an air expeller (20) for generating a flow of air while the device is being actuated, and a reservoir (30) containing a single dose of fluid, said reservoir (30) including a proximal axial end and a distal axial end, and being mounted in removable manner in said dispenser head (1) so that after the device has been actuated, the empty reservoir can be removed from said device and replaced by a new full reservoir, said air expeller (20) being adapted to return to its rest position so as to enable a new actuation with said new full reservoir, said device being **characterized in that** said dispenser head (1) includes a proximal perforator tip (11) that is adapted to perforate said proximal axial end of said reservoir (30), and **in that** it includes a movable perforator member (40) that is arranged to slide axially around said dispenser head (1) between a loading position and an actuation position, said movable perforator member (40) including a distal perforator tip (41) that is adapted to perforate the distal axial end of said reservoir (30) when said movable perforator member (40) is moved into its actuation position.

2. A device according to claim 1, wherein said reservoir (30) is symmetrical.

3. A device according to claim 1 or claim 2, wherein said reservoir (30) is a capsule.

4. A device according to any preceding claim, wherein said proximal and distal perforator tips (11, 41) are hollow, and each includes at least one axial opening (111, 411).

5. A device according to any preceding claim, wherein said distal perforator tip (41) forms an air inlet (31) in said reservoir (30), said air inlet (31) being connected to said air expeller (20), and said proximal perforator tip (11) forms a fluid outlet (32) in said reservoir (30), said fluid outlet (32) being connected to said dispenser orifice (10), such that while the device is being actuated, the flow of air generated by the air expeller (20) penetrates into said reservoir (30) through said air inlet (31), and drives said fluid out from said reservoir (30) through said fluid outlet (32), towards said dispenser orifice (10).

6. A device according to any preceding claim, wherein said proximal axial end of said reservoir (30) is perforated by said proximal perforator tip (11) while said reservoir (30) is being inserted into said dispenser head (1).

7. A device according to any preceding claim, wherein said dispenser head (1) includes a side window (4), arranged in the proximity of said proximal perforator tip (11), so as to enable said reservoir (30) to be inserted into said dispenser head (1).

8. A device according to claim 7, wherein said dispenser head (1) includes a movable or deformable portion (3), such as a spring blade, remote from said side window (4), so as to expel the empty reservoir out from said dispenser head (1), through said side window (4).

9. A device according to any preceding claim, wherein said air expeller includes a piston (21) that slides in an air chamber (22) between a rest position and a dispensing position, said air expeller (20) including an outlet member (51, 51') that enables the air in said air chamber (22) to be compressed during actuation, a return spring (23) being provided so as to urge said piston (21) resiliently towards its the rest position.

10. A device according to claim 9, wherein said piston (21), when in its rest position, co-operates in non-airtight manner with said air chamber (22), in such a manner that said air chamber (22) is in communication with the atmosphere in the rest position.

11. A device according to claim 9 or claim 10, wherein said outlet member of the air expeller (20) comprises a valve (51) that passes from a closed position to an open position once the air pressure in said air chamber (22) has reached a predetermined threshold.

12. A device according to claim 11, wherein said valve (51) comprises a slotted diaphragm that is closed in airtight manner in the closed position, and that deforms so as to open said slot once a predetermined pressure threshold has been reached.

13. A device according to claim 12, wherein, in its closed position, said diaphragm is convex towards said air chamber (22), and when the pressure threshold is reached, it inverts its shape and becomes concave, thereby making it possible for the slot to open.

14. A device according to claim 9 or claim 10, wherein said outlet member of the air expeller (20) comprises a ball (51') that is moved from a closed position to an open position once said piston (21) has reached a predetermined actuation stroke in said air chamber (22).

15. A device according to claim 14, wherein, after said piston (21) has passed through a predetermined fraction of the actuation stroke, a central rod (27) secured to said piston (21) co-operates with said ball (51') so as to expel it mechanically from its closed position.

16. A device according to claim 15, wherein said movable perforator member (40), when it is returned from its actuated position to its loading position, co-operates with said ball (51') so as to return it to its closed position.
